(19) Europäisches Patentamt
European Patent Office
Office européen des brevets

(11) Numéro de publication : **0 340 072 B1**

(12) **FASCICULE DE BREVET EUROPEEN**

(45) Date de publication du fascicule du brevet : **29.01.92 Bulletin 92/05**

(51) Int. Cl.$^5$ : **C07C 275/10**

(21) Numéro de dépôt : **89401048.7**

(22) Date de dépôt : **17.04.89**

(54) **Nouveau dérivé acrylique de l'urée.**

(30) Priorité : **29.04.88 FR 8805745**

(43) Date de publication de la demande : **02.11.89 Bulletin 89/44**

(45) Mention de la délivrance du brevet : **29.01.92 Bulletin 92/05**

(84) Etats contractants désignés : **AT BE CH DE ES FR GB GR IT LI LU NL SE**

(56) Documents cités : **FR-A- 2 580 635**

(73) Titulaire : **ATOCHEM**
**4 & 8, Cours Michelet La Défense 10**
**F-92800 Puteaux (FR)**

(72) Inventeur : **Garrigue, Roger**
**23, rue du Capitaine Escudié**
**F-31000 Toulouse (FR)**
Inventeur : **Lalo, Jack**
**116, avenue St Exupéry**
**F-31400 Toulouse (FR)**

(74) Mandataire : **Rieux, Michel et al**
**ATOCHEM Département Propriété Industrielle**
**4, Cours Michelet - La Défense 10 - Cedex 42**
**F-92091 Paris-La Défense (FR)**

EP 0 340 072 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un nouveau dérivé acrylique de l'urée.

On connaît déjà des dérivés acryliques de l'urée. Les composés acryliques utilisés comme produits de départ sont le plus souvent l'acrylamide et l'acrylonitrile. C'est ainsi qu'on a proposé la polycondensation de l'urée, du formol et de l'acrylamide en milieu faiblement alcalin en vue de préparer des produits méthylolés suivi de l'addition de styrène ou d'acrylate de méthyle (voir en particulier KOSTYUKOV et al-URSS 277, 550, 3 Juin 1970). On a aussi proposé la préparation de dérivés acryliques de l'urée à partir de méthacrylamide, de formol et d'urée (voir demande française 85.06106). On a aussi décrit un nouveau procédé de fabrication d'acrylamido méthylène-urée (voir demande 85.06103 déposé par la Demanderesse) qui consiste à faire réagir dans une première étape l'acrylamide sur le formol en milieu basique en vue d'obtenir la monométhylolacrylamide puis dans une seconde étape à faire réagir ce dernier composé sur de l'urée en milieu d'acide.

La présente invention concerne un nouveau dérivé acrylique de l'urée, le monométhylolacrylamidométhylène-urée.

Ce produit a la formule suivante :

$$CH_2 = CH - \underset{\underset{O}{\|}}{C} - NH - CH_2 - NH - \underset{\underset{O}{\|}}{C} - NH - CH_2OH$$

Ce produit se présente sous la forme d'un solide blanc qui change d'état physique entre 190 et 200°C.

Le nouveau dérivé acrylique de l'urée est préparé selon le mode opératoire suivant. Selon ce mode opératoire on met en oeuvre l'acrylamidométhylène urée obtenu selon le procédé de préparation décrit dans la demande française 85.06103 déposée par la Demanderesse. A ce dérivé de l'urée on ajoute de l'eau et une solution aqueuse de formol. La réaction est réalisée en présence d'un inhibiteur tel que éther méthylique de l'hydroquinone et de triéthylamine en chauffant pendant une heure environ à 60°C. Il se forme alors après refroidissement un précipité blanc que l'on sépare par filtration. Le produit est ensuite purifié par lavage à l'acétate d'éthyle, puis à l'acétone : il est ensuite recristallisé dans un mélange acétate d'éthyle éthanol.

Le nouveau dérivé acrylique objet de l'invention est préparé en mettant en oeuvre des quantités équimoléculaires d'acrylamidométhylène urée et de formol. Il convient particulièrement comme additifs dans les résines aminoplastes et les phénoplastes, en particulier ceux utilisés comme colles à bois. Dans les aminoplastes notamment il est mis en oeuvre sous forme de solution dans un précondensat urée-formol. Dans ce cas le monométhylolacrylamidométhylène urée en solution dans un précondensat urée-formol, est préparé selon les modes opératoires décrits dans le demande EP-A1-349355 déposée le même jour par la Demanderesse et intitulé "Nouveaux additifs convenant dans les résines aminoplastes". Ces modes opératoires consistent, soient à préparer le mométhylolacrylamidométhylène urée à partir d'acrylamide dans un précondensat urée-formol, soit à partir d'acrylamidométhylène-urée dans un précondensat urée-formol.

Les exemples suivants illustrent la présente invention :

### Exemple 1

Préparation du monométhylolacrylamidométhylène urée

Dans un réacteur de 250 ml, on introduit 11,34 g d'une solution aqueuse de formol à 37%, 40 g d'eau, 20 g d'acrylamidométhylène urée, 50 mg d'éther méthylique de l'hydroquinone et 0,2 g de triéthylamine. On chauffe le mélange réactionnel 1 heure à 60°C.

On obtient un mélange réactionnel, homogène et limpide, que l'on refroidit à la température ambiante. Il se forme alors un précipité blanc que l'on sépare par filtration, puis qu'on lave à l'acétate d'éthyle puis à l'acétone. Il est ensuite recristallisé dans un mélange acétate d'éthyle-éthanol (10/90).

| | |
|---|---|
| - quantité produit obtenu | m = 15,7 g |
| - rendement | = 65 % |

Le produit se présente sous l'aspect d'une poudre blanche par passage sur un banc Kofler, il se produit un changement d'état physique entre 190 et 200° celcius. L'analyse élémentaire réalisé sur le produit a donné les résultats suivants :

| | | | |
|---|---|---|---|
| - azote | : | 24,15 % | (théorie 24,27 %) |
| - carbone | : | 41,9 % | (théorie 41,6 %) |
| - hydrogène | : | 6,2 % | (théorie 6,36 %) |

Les spectres infra-rouge (figure 1) et RMN du proton (figure 2) et du carbone 13 (figure 3) dans le DMSO deutérié permettent de confirmer la structure du monométhylolacrylamidométhylène-urée.

Exemple 2

On prépare le monométhylolacrylamidométhylène urée en solution dans un précondensat urée-formol selon le mode opératoire décrit dans la demande déposée par la Demanderesse intitulée "Nouveau additifs convenant dans les résines aminoplastes".

Dans un réacteur on introduit 60 g d'acrylamidométhylène-urée pure, 120 mg de l'éther méthylique de l'hydroquinone et 350 g d'un précondensat urée-formol préparé à partir d'une solution de formol à 49% et d'une solution d'urée à 20%. On maintient le pH du milieu réactionnel à 7,5 et la réaction est conduite à 70°C jusqu'à solubilisation de l'acrylamidométhylène-urée.

Le dérivé méthylolée ainsi obtenu dans un précondensat urée-formol est utilisé comme additif dans une résine urée-formol.

## Revendications

1. Nouveau dérivé acrylique de l'urée caractérisé par le fait que ce dérivé est le monométhylolacrylamidométhylène-urée de formule :

$$CH_2 = CH - \underset{\underset{O}{\|}}{C} - NH - CH_2 - NH - \underset{\underset{O}{\|}}{C} - NH - CH_2OH$$

2. Procédé de préparation de monométhylolacrylamidométhylène-urée selon 1 caractérisé en ce qu'on fait réagir de l'acrylamidométhylène-urée avec du formol en présence d'un inhibiteur.

3. Procédé de préparation de monométhylolacrylamidométhylène-urée selon 1 et 2 caractérisé en ce qu'on utilise des quantités équimoléculaires d'acrylamidométhylène urée et de formol.

## Patentansprüche

1. Neue Acrylharnstoff-Verbindung, dadurch gekennzeichnet, daß die Verbindung Monomethylolacrylamidomethylen-Harnstoff der Formel

$$CH_2 = CH - \underset{\underset{O}{\|}}{C} - NH - CH_2 - NH - \underset{\underset{O}{\|}}{C} - NH - CH_2OH$$

ist.

2. Verfahren zur Herstellung des Monomethylolacrylamidomethylen-Harnstoffs nach Anspruch 1, dadurch gekennzeichnet, daß man Acrylamidomethylen-Harnstoff mit Formol in Gegenwart eines inhibitors reagieren läßt.

3. Verfahren zur Herstellung des Monomethylolacrylamidomethylen-Harnstoffs nach Anspruch 1 und 2, dadurch gekennzeichnet, daß man äquimolekulare Mengen von Acrylamidomethylen-Harnstoff und Formol einsetzt.

## Claims

1. New acrylic derivative of urea, characterized in that this derivative is monomethylolacrylamidomethyleneurea of formula :

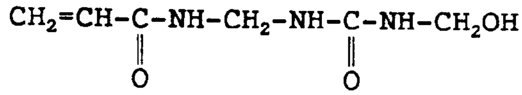

2. Process for preparing monomethylolacrylamidomethyleneurea according to 1, characterized in that acrylamidomethyleneurea is reacted with formaldehyde in the presence of an inhibitor.

3. Process for preparing monomethylolacrylamidomethyleneurea according to 1 and 2, characterized in that equimolecular quantities of acrylamidomethyleneurea and formaldehyde are used.

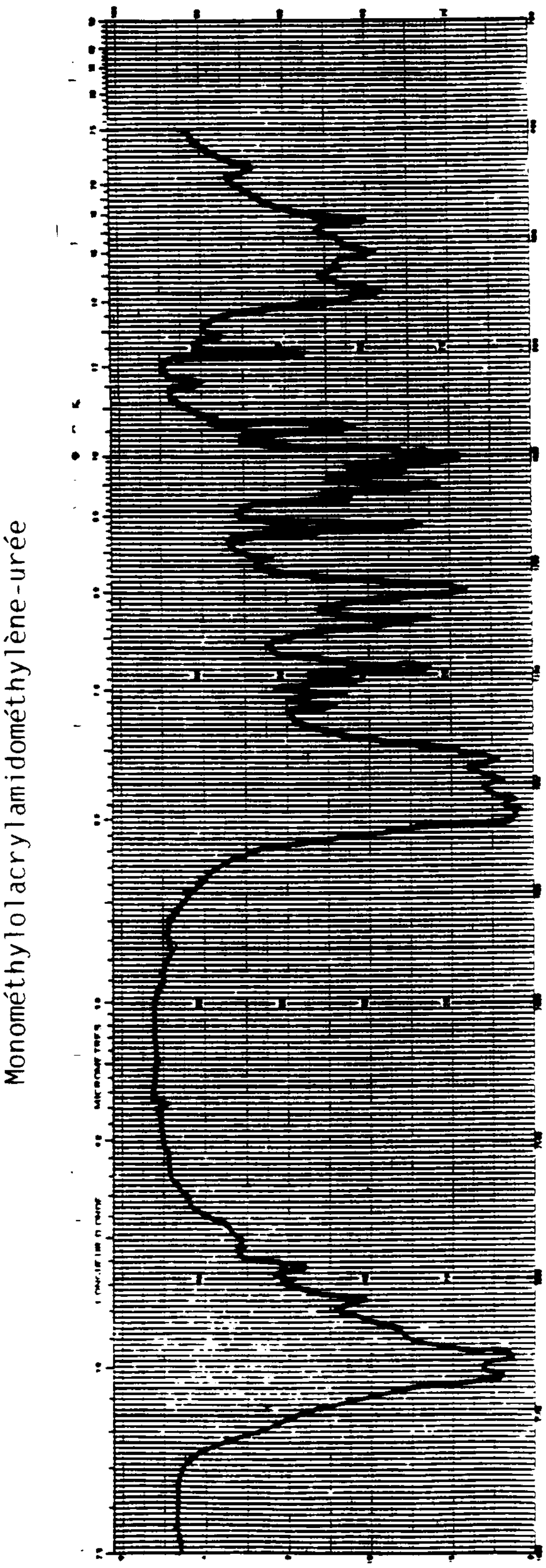


Figure 1

N-KETHYLOL ACRYLAMIDO METHYLENE UREE

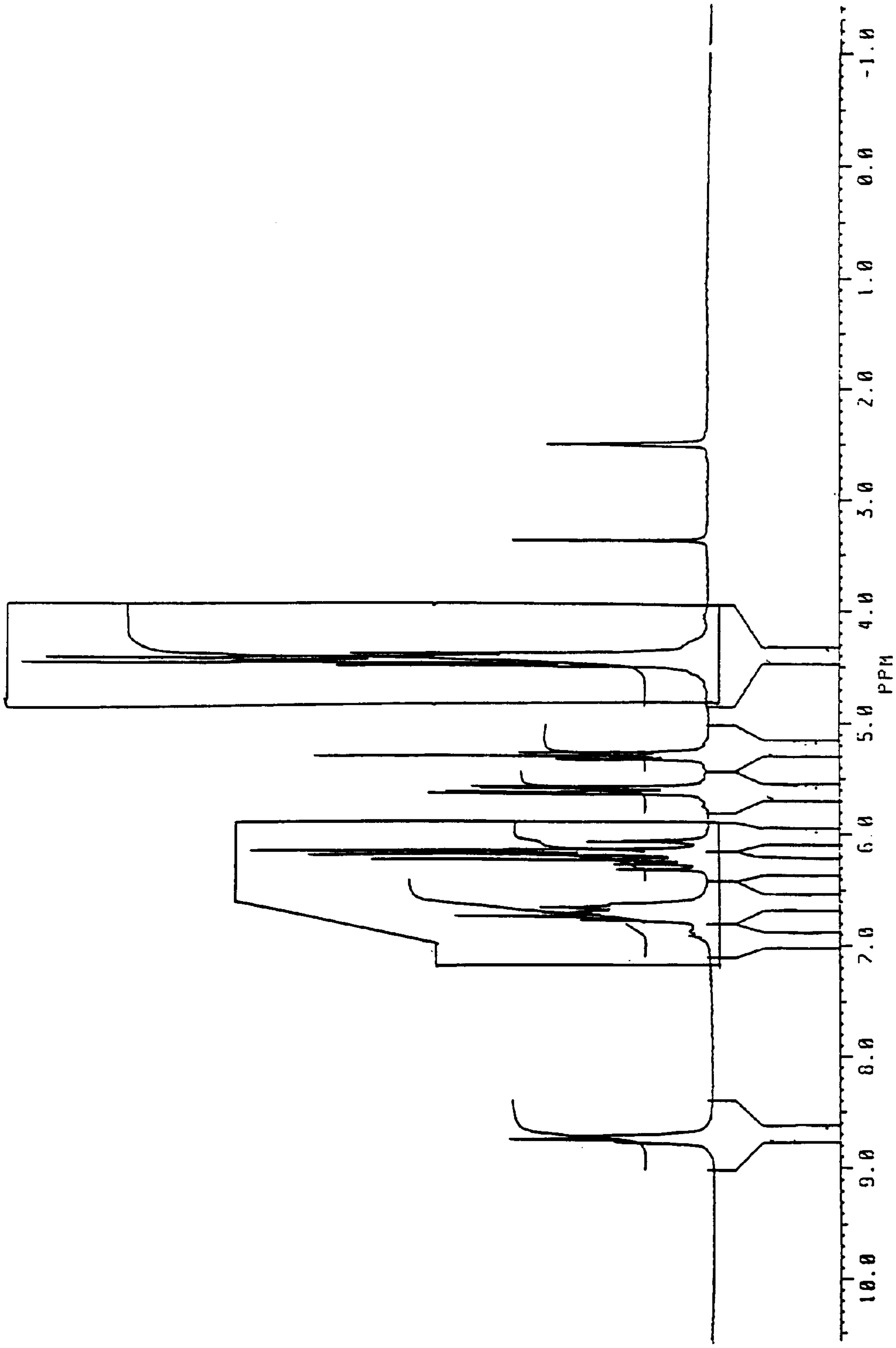


Figure 2

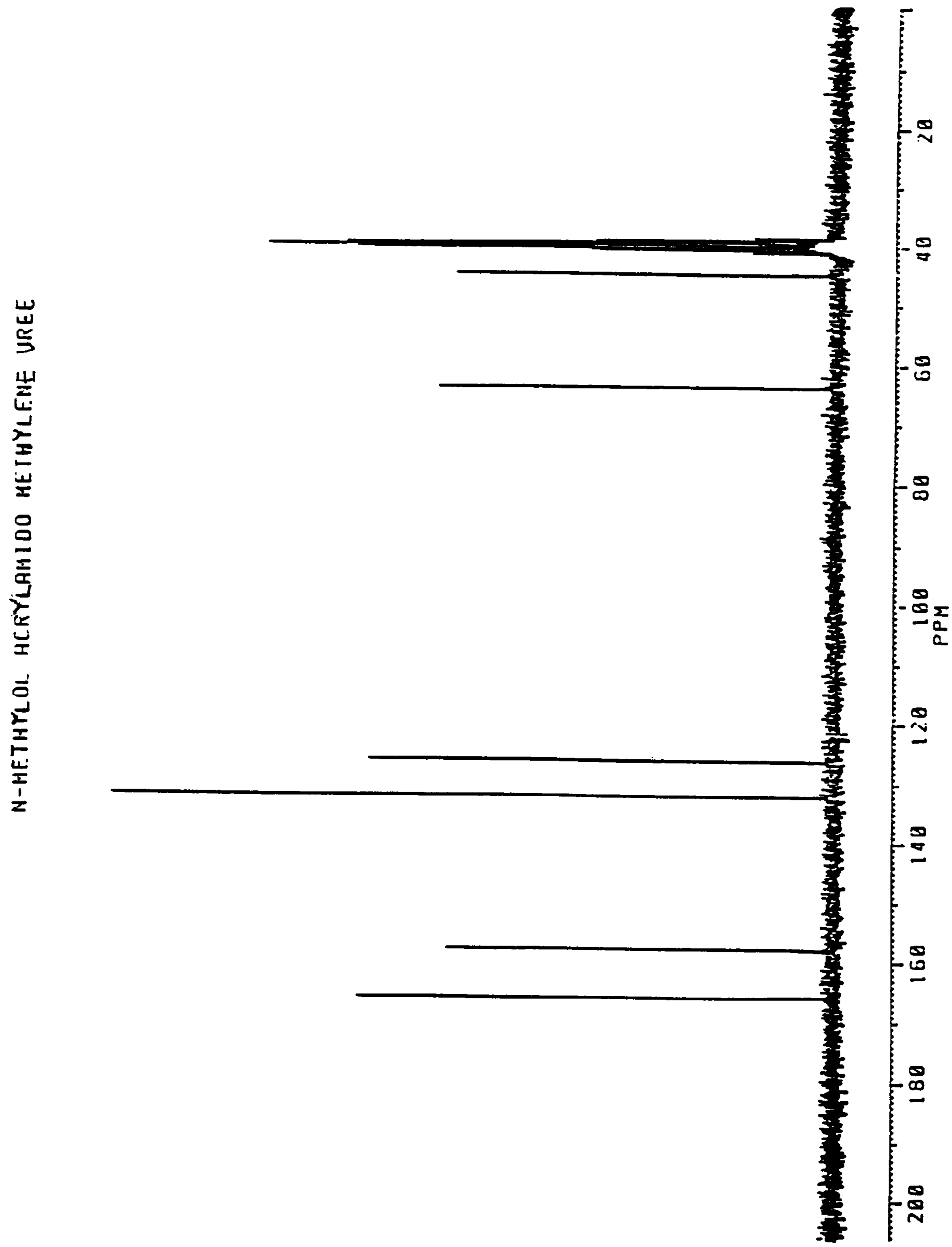
N-METHYLOL ACRYLAMIDO METHYLENE UREE
20
40
60
80
100
120
140
160
180
200
PPM

Figure 3